Europäisches Patentamt

European Patent Office

Office européen des brevets

(11) Numéro de publication: **0 223 658**
**B1**

(12) **FASCICULE DE BREVET EUROPEEN**

(45) Date de publication du fascicule du brevet:
**01.08.90**

(21) Numéro de dépôt: **86402286.8**

(22) Date de dépôt: **14.10.86**

(51) Int. Cl.⁵: **A61B 6/00**

(54) Appareil de radiologie à faisceau de rayons X de direction variable.

(30) Priorité: **18.10.85 FR 8515505**

(43) Date de publication de la demande:
**27.05.87 Bulletin 87/22**

(45) Mention de la délivrance du brevet:
**01.08.90 Bulletin 90/31**

(84) Etats contractants désignés:
**DE NL**

(56) Documents cités:
**FR-A- 2 137 892**
**GB-A- 2 098 440**

(73) Titulaire: **GENERAL ELECTRIC CGR S.A., 100, rue Camille-Desmoulins, F-92130 Issy les Moulineaux(FR)**

(72) Inventeur: **Chambron, Edmond, THOMSON-CSF SCPI 19, avenue de Messine, F-75008 Paris(FR)**

(74) Mandataire: **Ballot, Paul Denis Jacques, Cabinet Ballot-Schmit 7, rue le Sueur, F-75116 Paris(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

L'invention est relative à un appareil d'observation du corps humain aux rayons X.

Pour l'examen de certaines parties du corps humain, notamment les vaisseaux sanguins, on fait appel à un faisceau de rayons X de direction variable de manière à pouvoir observer ces parties sous diverses incidences.

Ainsi pour examiner une zone quasi ponctuelle du corps le faisceau de rayons X peut tourner - de préférence selon toutes les directions possibles - autour d'un point géométrique, appelé "isocentre", qui est attaché à l'appareil d'exploration et qu'on fait coïncider avec la zone à examiner.

Par ailleurs il est nécessaire que l'isocentre soit aussi déplaçable selon une courbe de forme quelconque, par exemple pour examiner un vaisseau sanguin.

Un appareil assurant ces fonctions est appelé un statif d'exploration à isocentre mobile. Il comporte une source de rayons X et un détecteur fixes l'un par rapport à l'autre; pour la commodité de l'exposé on se réfèrera dans ce qui suit à un vecteur $\overrightarrow{RX}$ dont la direction est celle du faisceau produit par la source vers le détecteur.

En plus de la mobilité du faisceau un tel appareil d'exploration doit satisfaire aux exigences suivantes : il faut pouvoir passer d'une position d'exploration à une autre en un temps relativement restreint sans avoir à arrêter l'exploration; l'encombrement doit être modéré afin que la salle d'examen ne soit pas de dimensions trop importantes; de plus il peut être souhaitable de faire varier le grandissement, c'est-à-dire de faire varier la position de la zone à observer sur le vecteur $\overrightarrow{RX}$. En outre les mouvements du praticien chargé de l'examen ne doivent pas être entravés.

Un appareil réalisant ces fonctions comprend un support, habituellement un arceau, pour l'ensemble source-détecteur et permettant la rotation du vecteur $\overrightarrow{RX}$ dans un plan. Ce support est solidaire d'un bras pouvant tourner autour d'un axe horizontal distinct de celui autour duquel peut tourner le vecteur $\overrightarrow{RX}$ dans le plan. Ce bras est lui-même solidaire d'une colonne verticale le long de laquelle il peut se déplacer. Par ailleurs, l'appareil comporte une table sur laquelle repose le patient à examiner.

Dans le brevet US 4 481 656 est décrit un appareil de ce type qui remplit les conditions indiquées ci-dessus.

Mais cet appareil est complexe et onéreux.

L'invention remédie à cet inconvénient.

L'invention se rapporte à un appareil d'observation du corps humain aux rayons X, notamment pour l'examen des vaisseaux sanguins d'un patient comprenant:

- une table horizontale pour recevoir le corps du patient à examiner, ladite table horizontale étant au moins mobile suivant une direction longitudinale,

- une source de rayons X fournissant un faisceau de rayons X ($\overrightarrow{RX}$) et étant supportée par une extrémité d'un arceau,

- un détecteur de rayons X supporté par l'autre extrémité de l'arceau,

- un bras pouvant tourner autour d'un axe horizontal parallèle à la direction longitudinale de la table et portant ledit arceau,

- une colonne supportant ledit bras tournant et permettant un déplacement vertical dudit bras, ladite colonne étant mobile suivant une direction (Y) perpendiculaire à la direction longitudinale de la table, caractérisé

- en ce que ledit arceau est semi-circulaire et peut coulisser selon une trajectoire circulaire dont l'axe est perpendiculaire à la direction longitudinale de la table,

- en ce que la source et le détecteur sont chacun à l'extrémité d'un bras en saillie par rapport à une face latérale de l'arceau,

- en ce que le faisceau de rayons X ($\overrightarrow{RX}$) peut tourner autour d'un isocentre de manière à permettre l'observation d'une zone du patient sous une incidence variable par la combinaison des mouvements de rotation dudit bras, dudit arceau semi-circulaire et des mouvements de translation verticale dudit bras sur la colonne verticale et de translation horizontale (Y) de ladite colonne verticale, et

- en ce que ledit isocentre est mobile suivant une courbe quelconque par un mouvement de translation longitudinale de ladite table combiné avec les mouvements de translation verticale dudit bras sur la colonne verticale et de translation horizontale (Y) de ladite colonne verticale.

Les différents mouvements combinés sont commandables à l'aide d'un calculateur.

L'appareil comporte, en outre, des moyens de mise en mémoire de trajets de l'isocentre pour la commande automatique des mouvements en vue d'obtenir des déplacements de l'isocentre selon ces trajets mis en mémoire.

D'autres caractéristiques et avantages de l'invention apparaîtront avec la description de certains de ses modes de réalisation, celle-ci étant effectuée en se référant aux dessins ci-annexés sur lesquels:

- la figure 1 est un schéma d'un appareil selon l'invention,
- la figure 2 est une vue selon la flèche f de la figure 1,
- la figure 3 est une vue selon la flèche F de la figure 1,
- la figure 4 est une vue en perspective de l'appareil représenté sur les figures 1 à 3, et
- la figure 5 est une vue analogue à celle de la figure 4 mais pour une autre position d'examen.

L'exemple que l'on va décrire en relation avec les figures se rapporte à un appareil d'examen des vaisseaux sanguins appelés statif d'exploration vasculaire.

Un tel appareil comprend une source de rayons X 10 émettant un faisceau de rayons X vers un ensemble détecteur 11 associé à des moyens d'imagerie (non représentés) permettant la photographie ou la reproduction d'une image sur un écran de visualisation tel que celui d'un tube à rayons cathodiques et, éventuellement, la mémorisation dans un ordinateur.

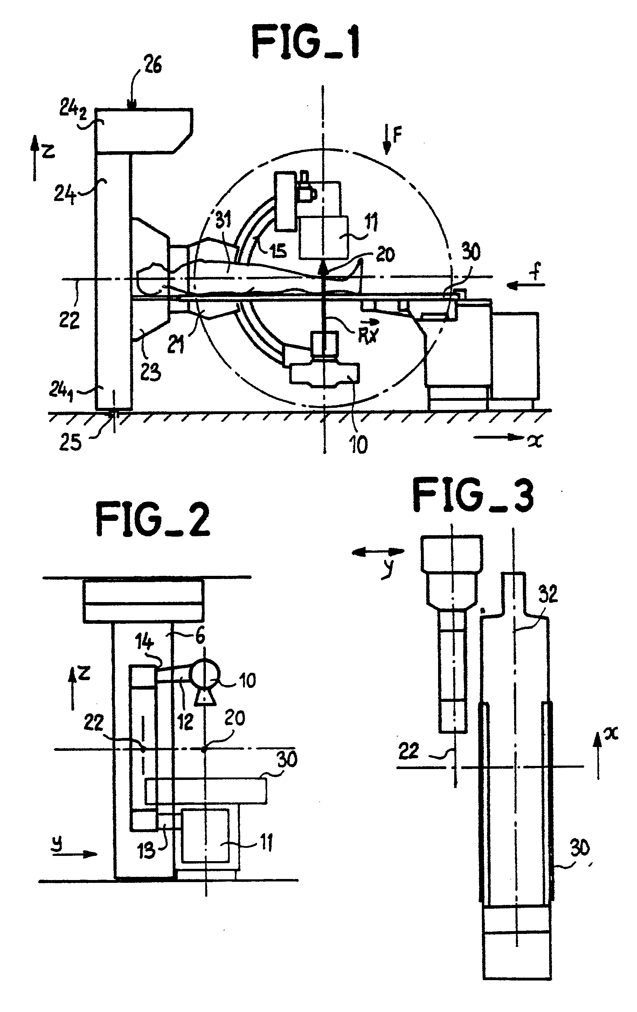

La source 10 et le détecteur 11 sont chacun solidaires de bras parallèles, respectivement 12 et 13, qui sont en saillie de la face latérale 14 d'un support 15 en forme d'arc de cercle ou arceau. Le support 15 peut coulisser dans une glissière ménagée à l'extrémité d'un bras support 21. Le faisceau lumineux produit par la source 10, qui est représenté par le vecteur $\vec{RX}$ sur la figure 1, peut ainsi tourner autour d'un point 20 dans un plan parallèle au plan de l'arceau 15.

Par ailleurs le bras support 21 peut tourner autour d'un axe horizontal 22 et ce bras 21 s'appuie sur un autre support 23 pouvant coulisser le long d'une colonne verticale 24.

La colonne 24 est guidée pour pouvoir se déplacer horizontalement dans une direction perpendiculaire à l'axe 22. A cet effet la partie inférieure $24_1$ de cette colonne coopère, par exemple grâce à des roulettes, avec des rails 25 au plancher et sa partie supérieure $24_2$ coopère, également par exemple grâce à des roulettes, avec des rails 26 au plafond.

L'appareil comporte aussi une table à plateau horizontal 30 destinée à recevoir le patient 31. Le plateau 30 présente un axe longitudinal 32 parallèle à l'axe 22. Ce plateau 30 est déplaçable selon cet axe 32, le patient 31 présentant sa tête dirigée vers la colonne 24.

La source 10 et le détecteur 11 étant solidaires d'un même support 15 les mouvements du vecteur $\vec{RX}$ sont ceux dudit support. Ainsi ce vecteur $\vec{RX}$ peut tourner autour du point 20 selon deux degrés de liberté : une rotation dans un plan parallèle à celui de l'arceau 15, autour du point 20, et une rotation correspondant au pivotement autour de l'axe 22. Le point 20 constitue l'isocentre, c'est-à-dire le point autour duquel doit tourner le vecteur $\vec{RX}$ pour l'examen d'une zone quasi ponctuelle du patient 31. Du fait des deux degrés de liberté la zone à examiner peut être observée sous n'importe quel angle.

Pour l'examen d'une zone on fait coïncider le centre 20 avec cette zone du patient. Etant donné que l'isocentre 20 n'est pas situé sur l'axe 22, quand on fait tourner le support 21 autour de l'axe 22 cet isocentre se déplace si le support 23 et la colonne 24 restent au même emplacement au cours de la rotation; ainsi pour maintenir la coïncidence entre le centre 20 avec la zone du patient à examiner il est nécessaire, quand on fait tourner le vecteur $\vec{RX}$ de déplacer le support 23 le long de la colonne 24 et de déplacer la colonne 24 sur ses rails. Autrement dit pour maintenir l'isocentre 20 dans une position fixe par rapport au patient il faut, lorsqu'on fait tourner le vecteur $\vec{RX}$ autour de l'axe 22, conjuguer cette rotation avec un déplacement vertical et un déplacement horizontal selon la direction des rails. Cette conjugaison de mouvements est effectuée de façon automatique par des moyens de commande non représentés, un calculateur agissant sur les moteurs d'entraînement des diverses parties mobiles de l'appareil.

L'isocentre 20 est déplaçable selon trois degrés de liberté : un premier degré de liberté est la direction X de l'axe 32 de la table; un second degré de liberté est le déplacement selon la direction horizontale Y, celle des rails 25, 26, perpendiculaire à la direction X; le troisième degré de liberté est la direction verticale Z par le déplacement du support 23 sur la colonne 24.

Il est ainsi possible de déplacer l'isocentre 20 selon une courbe de forme quelconque si l'on conjugue des mouvements selon les directions X, Y et Z. Ces mouvements sont aussi commandés par calculateur.

Toutefois les mouvements de l'isocentre sont, dans l'exemple, limités par le déplacement relatif de la colonne 24 selon l'axe Y et du plateau 30 selon l'axe X. Pour la même raison certains mouvements du vecteur $\vec{RX}$ ne sont pas possibles.

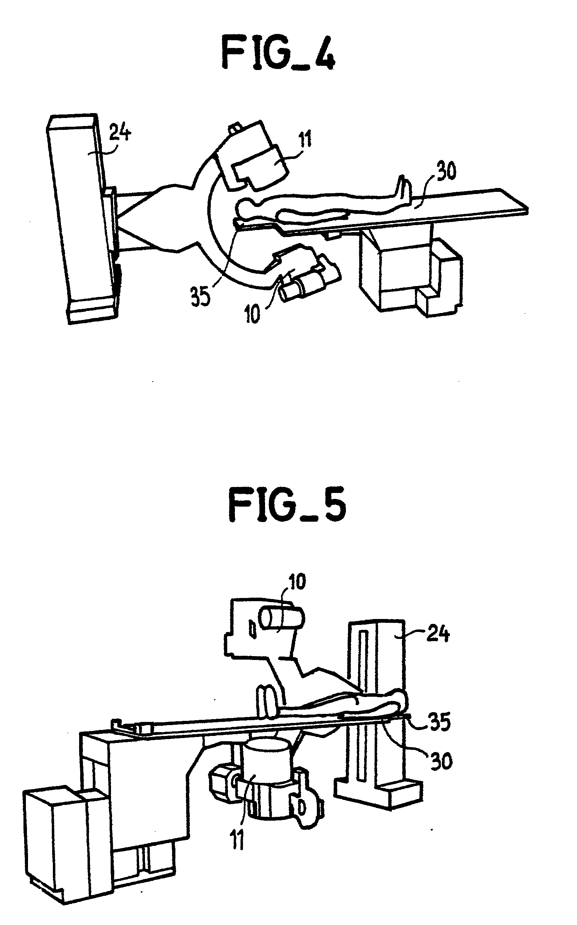

Par exemple dans la position représentée sur la figure 5, où le plateau 30 a son extrémité antérieure 35 au voisinage de la colonne 24, il n'est pas possible, sans déplacer le plateau 30, de tourner le vecteur $\vec{RX}$ de 180°, c'est-à-dire d'inverser les positions de la source et du détecteur. On pourrait obtenir ce résultat en déplaçant le plateau 30 c'est-à-dire en interrompant momentanément la visualisation. Mais lorsque le plateau 30 est dans la position représentée sur la figure 5 l'appareil est utilisé pour l'examen de la partie inférieure du corps, en-dessous de l'abdomen. Or, pour de tels examens il n'est en général pas nécessaire de disposer d'incidences s'écartant beaucoup de la perpendiculaire au plateau 30. Ainsi la limitation mentionnée n'est pas gênante.

Par contre cette limitation n'existe pas pour l'examen de la partie supérieure du corps, au-dessus de l'abdomen. En effet dans ce cas l'appareil est dans la position représentée sur la figure 4, c'est-à-dire que l'extrémité 35 du plateau 30 est éloignée de la colonne 24 et, dans ce cas, il est possible de conférer une orientation quelconque au vecteur $\vec{RX}$ sans interrompre l'examen, ce qui est utile notamment pour l'examen de la zone du coeur et l'examen de la tête.

En d'autres termes encore on pourrait considérer que la simplicité de réalisation de l'appareil de l'invention est obtenue au détriment de ses performances; mais il n'en est rien car la limitation de ses possibilités pour l'examen de la partie inférieure du corps est sans conséquence particulière pour l'utilisateur de l'appareil.

Il est possible de faire suivre au point 20 une courbe quelconque afin d'examiner, par exemple, un vaisseau sanguin suivant une ligne gauche. Cette visualisation peut même être effectuée de façon automatique. Dans ce dernier cas on détermine tout d'abord par radioscopie, c'est-à-dire par visualisation directe de l'image, le trajet à suivre que l'on met au fur et à mesure en mémoire du calculateur. Ensuite le calculateur commande les mouvements des divers organes de l'appareil pour suivre le trajet ainsi mis en mémoire de façon, par exemple, à mettre en mémoire les images du vaisseau sanguin à une vitesse déterminée d'observation.

Il est également à noter qu'il est possible de faire varier le grandissement de l'image par un déplacement selon les seules directions Z et Y.

L'appareil de l'invention permet un examen en continu d'un patient de la tête aux pieds à condition, comme on l'a déjà vu ci-dessus, que pour la partie inférieure du corps l'incidence du faisceau de rayons X ne soit pas trop éloignée de la normale au plateau 30.

L'appareil selon l'invention est d'une grande simplicité, son encombrement est restreint et ces avantages ne sont pas obtenus au détriment des performances.

## Revendications

1. Appareil d'observation du corps humain aux rayons X, notamment pour l'examen des vaisseaux sanguins d'un patient comprenant:
   - une table horizontale (30) pour recevoir le corps du patient à examiner, ladite table horizontale étant au moins mobile suivant une direction longitudinale (32),
   - une source de rayons X (10) fournissant un faisceau de rayons X ($\overrightarrow{RX}$) et étant supportée par une extrémité d'un arceau (15),
   - un détecteur de rayons X (11) supporté par l'autre extrémité de l'arceau (15),
   - un bras (21) pouvant tourner autour d'un axe horizontal (22) parallèle à la direction longitudinale (32) de la table et portant ledit arceau (15),
   - une colonne (24) supportant ledit bras tournant (21) et permettant un déplacement vertical dudit bras (21), ladite colonne étant mobile suivant une direction (Y) perpendiculaire à la direction longitudinale (32) de la table (30), caractérisé
   - en ce que ledit arceau (15) est semi-circulaire et peut coulisser selon une trajectoire circulaire dont l'axe est perpendiculaire à la direction longitudinale (32) de la table (30),
   - en ce que la source (10) et le détecteur (11) sont chacun à l'extrémité d'un bras (12, 13) en saillie par rapport à une face latérale (14) de l'arceau (15),
   - en ce que le faisceau de rayons X ($\overrightarrow{RX}$) peut tourner autour d'un isocentre (20) de manière à permettre l'observation d'une zone du patient sous une incidence variable par la combinaison des mouvements de rotation dudit bras (21), dudit arceau semi-circulaire (15) et des mouvements de translation verticale (Z) dudit bras (21) sur la colonne verticale (24) et de translation horizontale (Y) de ladite colonne verticale (24), et
   - en ce que ledit isocentre (20) est mobile suivant une courbe quelconque par un mouvement de translation longitudinale de ladite table (30) combiné avec les mouvements de translation verticale (Z) dudit bras (21) sur la colonne verticale (24) et de translation horizontale (Y) de ladite colonne verticale (24).

2. Appareil selon la revendication 1, caractérisé en ce qu'il comporte un calculateur pour la commande des déplacements de l'isocentre (20) et de l'orientation du faisceau de rayon X ($\overrightarrow{RX}$).

3. Appareil selon la revendication 2, caractérisé en ce qu'il comporte des moyens de mise en mémoire de trajets de l'isocentre (20) pour la commande automatique des mouvements en vue d'obtenir des déplacements de l'isocentre (20) selon ces trajets mis en mémoire.

## Claims

1. An apparatus for the observation of a human body using x-rays, more especially for the examination of blood vessels of a patient, comprising:
   - a horizontal table (30) in order to support the body of the patient to be examined, the said horizontal table being at least able to be moved in a horizontal direction (32),
   - a source of x-rays (10) supplying a beam of x-rays ($\overrightarrow{RX}$) and supported by one extremity of an arch (15),
   - an x-ray detector (11) supported by the other extremity of the arch (15),
   - an arm (21) able to turn about a horizontal axis (22) parallel to the longitudinal direction (32) of the table and bearing the said arch (15),
   - a column (24) supporting the said turning arm (21) and allowing a vertical displacement of the said arm (21), the said column being able to move along a direction (Y) perpendicular to the longitudinal direction (32) of the table (30), characterized in that
   - the said arch (15) is semicircular and is able to slide along a circular path, whose axis is perpendicular to the longitudinal direction (32) of the table (30),
   - in that the source (10) and the detector (11) are each placed at the extremity of an arm (12 and 13) projecting in relation to a lateral face (14) of the arch (15),
   - in that the x-ray beam ($\overrightarrow{RX}$) is able to turn about an isocenter (20) in such a manner as to permit the observation of a zone of the patient at a variable incidence by the combination of movements of rotation of the said arm (21), of the said semicircular arch (15) and of the movements in vertical translation (Z) of the said arm (21) on the vertical column (24) and horizontal translation (Y) of the said vertical column (24), and
   - in that the said isocenter (20) is able to move along a curve with a movement of longitudinal translation of the said table (30) combined with the movements of vertical translation (Z) of the said arm (21) on the vertical column (24) and of horizontal translation (Y) of the said vertical column (24).

2. The apparatus as claimed in claim 1, characterized in that it comprises a calculator in order to control the displacements of the isocenter (20) and of the orientation of the beam of x-rays.

3. The apparatus as claimed in claim 2, characterized in that it comprises means for storing the paths of the isocenter (20) for the automatic command of movements in order to produce displacements of the isocenter (20) along the stored paths.

**Patentansprüche**

1. Gerät zur Beobachtung des menschlichen Körpers mit Röntgen-Strahlen, insbesondere für die Untersuchung der Blutgefäße eines Patienten, mit:
- einem waagerechten Tisch (30) zum Aufnehmen des Körpers des zu untersuchenden Patienten, wobei dieser waagerechte Tisch wenigstens in einer Längsrichtung (32) beweglich ist,
- einer Röntgen-Strahlenquelle (10), die ein Röntgenstrahlen-Bündel ($\vec{RX}$) liefert und von einem Ende eines Bogens (15) getragen wird,
- einem Röntgenstrahlen-Detektor (11), der von dem anderen Ende des Bogens (15) getragen wird,
- einem Arm (21), der sich um eine waagerechte Achse (22) drehen kann, zu der Längsrichtung (32) des Tisches parallel ist und den genannten Bogen (15) trägt,
- einer Säule (24), die den genannten drehbaren Arm (21) trägt und eine senkrechte Verschiebung desselben ermöglicht, wobei diese Säule in einer zu der Längsrichtung (32) des Tisches (30) senkrechten Richtung (Y) beweglich ist, dadurch gekennzeichnet, daß:
- der Bogen (15) halbkreisförmig ist und entlang einer kreisförmigen Strecke gleiten kann, deren Achse zu der Längsrichtung (32) des Tisches (30) senkrecht ist,
- die Quelle (10) sowie der Detektor (11) sich jeweils an einem Ende eines Armes (12, 13) befinden, der in bezug auf eine Seitenfläche (14) des Bogens (15) herausragt,
- das Röntgenstrahlen-Bündel ($\vec{RX}$) um ein Isozentrum (20) herum drehbar ist, so daß die Beobachtung einer Zone des Patienten unter einem Einfallswinkel möglich ist, der durch die Kombination der Drehbewegungen des Armes (21), des halbkreisförmigen Bogens (15) und der senkrechten Translationsbewegungen (Z) des Armes (21) auf der senkrechten Säule (24) sowie der waagerechten Translation (Y) der genannten senkrechten Säule (24) variabel ist, und
- daß das Isozentrum (20) entlang einer beliebigen Kurve durch eine longitudinale Translationsbewegung dieses Tisches (30) in Kombination mit den senkrechten Translationsbewegungen (Z) des Armes (21) auf der senkrechten Säule (24) und der waagerechten Translation (Y) der senkrechten Säule (24) beweglich ist.

2. Gerät nach Anspruch 1, dadurch gekennzeichnet, daß es einen Rechner für die Steuerung der Verschiebungen des Isozentrums (20) sowie der Orientierung des Röntgenstrahlen-Bündels umfaßt.

3. Gerät nach Anspruch 2, dadurch gekennzeichnet, daß es Mittel für die Speicherung der Bewegungsstrecken des Isozentrums (20) für die automatische Steuerung der Bewegungen umfaßt, um Verschiebungen des Isozentrums (20) entlang diesen gespeicherten Bewegungsstrecken zu erhalten.

# FIG_1

# FIG_2

# FIG_3

# FIG_4

# FIG_5